# EUROPEAN PATENT SPECIFICATION

(11) **EP 1 706 132 B1**
(45) Date of publication and mention of the grant of the patent: **23.03.2011**
(21) Application number: 04808335.6
(22) Date of filing: 07.12.2004
(51) Int. Cl.: A61K 38/16

(54) **A COMPOSITION FOR CARTILAGE THERAPEUTICS AND USING METHOD THEREOF**
ZUSAMMENSETZUNG FÜR KNORPEL-THERAPEUTIKA UND ANWENDUNGSVERFAHREN DAFÜR
COMPOSITION POUR LA THERAPEUTIQUE DU CARTILAGE ET SON PROCEDE D'UTILISATION

(30) Priority: 23.12.2003 KR 2003095340
(43) Date of publication of application: 04.10.2006
(73) Proprietor: Sewon Cellontech Co., Ltd., Youngdeungpo-Gu, Seoul 150-712 (KR)
(72) Inventor: CHANG, Cheong-Ho, Seoul 137-040 (KR); KO, Chang-Kwon, 110-2002 Dongbu centrebill Apt, Seoul 136-113 (KR); JANG, Jae-Deog, Seoul 139-222 (KR); LEE, Eun-Young, Seoul 140-190 (KR); CHOI, Jeong-Yong, Seoul 122-070 (KR)
(74) Representative: Merkle, Gebhard
(86) International application number: PCT/KR2004/003204
(87) International publication number: WO 2005/060987

(56) References cited:
- EP-A- 1 535 633
- WO-A-01/85225
- WO-A-03/007873
- WO-A1-92/13535
- DE-A1- 19 949 290
- OCHI M ET AL: "Current concepts in tissue engineering technique for repair of cartilage defect" ARTIFICIAL ORGANS, BLACKWELL SCIENTIFIC PUBLICATIONS, INC., BOSTON, US, vol. 25, no. 3, 1 March 2001 (2001-03-01), pages 172-179, XP002272583 ISSN: 0160-564X
- BRITTBERG M ET AL: "TREATMENT OF DEEP CARTILAGE DEFECTS IN THE KNEE WITH AUTOLOGOUS CHONDROCYTE TRANSPLANTATION" NEW ENGLAND JOURNAL OF MEDICINE, THE, MASSACHUSETTS MEDICAL SOCIETY, WALTHAM, MA, US, vol. 331, no. 14, 1 October 1994 (1994-10-01), pages 889-895, XP000891635 ISSN: 0028-4793
- ERGGELET CHRISTOPH ET AL: "The arthroscopic implantation of autologous chondrocytes for the treatment of full-thickness cartilage defects of the knee joint." ARTHROSCOPY, vol. 19, no. 1, January 2003 (2003-01), pages 108-110, XP002527897 ISSN: 0749-8063
- PASSARETTI D. ET AL: 'Cultured chondrocytes produce injectable tissue-engineered cartilage in hydrogel polymer' TISSUE ENG. vol. 7, no. 6, 2001, pages 805 - 815, XP008049896
- PERETTI G.M. ET AL: 'A biochemical analysis of an engineered cell-scaffold implant for artilage repair' ANN PLAST SURG. vol. 46, no. 5, 2001, pages 533 - 537, XP008049897
- PERETTI G.M. ET AL: 'Cell-bases tissue-engineered allogenic for cartilage repair' TISSUE ENG. vol. 6, no. 5, 2000, pages 567 - 576, XP008049895

## Description

### [Technical Field]

The present invention relates to a cartilage therapeutic composition, capable of being clinically transplanted to articulatio genu (knee joint) or ankle joints, in particular clinically significant, symptomatic cartilage defects region of the femoral condyle (medial, lateral, or trochlear) and bone cartilage defect regions of the talus (anklebone), in human or animal hosts, and a method of using the same.

### [Background Art]

Generally, cartilage tissues constituting vertebral joints, once damaged, cannot be regenerated to their original state in vivo.

Where articular cartilage tissues are damaged, normal daily activity is limited with severe pain. Initial articular cartilage tissue damage progresses to a chronic state, called fatal degenerative arthritis, which interferes with normal life.

Meanwhile, in order to treat articular cartilage damage, presently, one method of treatment is to grind the damaged articular surface thus rendering it soft, or another method is to induce micro fracture or make a small hole at the articular surface.

However, such methods have disadvantages such as recurrence of symptoms within several weeks thus making it impossible to return to a normal lifestyle. In addition, fibrous tissues prepared via such treatment methods completely qualitatively different from normal cartilage tissues.

Further, where the articular cartilage tissues are significantly damaged, regeneration of cartilage tissues becomes impossible. Therefore, when cartilage tissues are incapable of being regenerated due to a severely damaged state thereof, given conventional treatment methods, the damaged articular cartilage must be completely removed and replaced with a metal or plastic artificial joint.

Up to now, such an artificial joint replacement technique is known as the only method for treating cartilage damage that is too severe to be treated using regeneration techniques.

In the US alone, 300,000 of the above-mentioned artificial knee joint replacements are conducted per year.

However, the artificial articular cartilage is incapable of permanent use, as it has a service life of about 10 years as the device. Additionally, the price of the device is more than 4000 dollars thus imposing a heavy economic burden on patients.

Further, the frequency of complication recurrence for such artificial articular cartilage is excessively high, thus being inapplicable to active young people.

Recently, in order to treat damaged articular cartilage, a great deal of attention has been directed to autologous chondrocyte transplantation as a new treatment method based on tissue engineering.

This technique was first investigated in the Hospital for Joint Disease, in New York, USA, and further developed by the University of Gothenburg and Sahlgrenska University Hospital in Sweden.

Autologous chondrocyte transplantation is a method for treating damaged cartilage of a patient's joint, involving collecting cartilage from a lesser-weight bearing area of the patient suffering from damaged cartilage of knee joints followed by separation of chondrocytes, culturing the separated chondrocytes to obtain an amount of chondrocytes sufficient for treating cartilage defect regions, and then transplanting the cultured chondrocytes into cartilage defect regions of the patients to restore damaged articular cartilage.

Performance of this autologous chondrocyte transplantation was verified as a fundamental joint treatment based on various clinical results, but necessarily involves collecting a periosteum upon transplanting chondrocyte therapeutic agent and after transplanting into an affected part, injecting the chondrocyte therapeutic agent.

In addition, the autologous chondrocyte transplantation has various disadvantages such as large incision of the knee associated with periosteum collection, leakage possibility of the chondrocyte therapeutic agent due to incomplete suture between surgical site and periosteum, excessive time consumption in suturing between the periosteum and articular defect region, and the like.

### [Disclosure]

### [Technical Problem]

Therefore, the present invention has been made in view of the above problems, and it is an object of the present invention to provide a cartilage therapeutic composition, capable of solving difficulty due to periosteum collection, suture, incomplete suture between the periosteum and transplanted site, and large incision and the time required for surgery, upon transplanting cartilage therapeutic agent, and a method of using the same.

It is another object of the present invention to provide a cartilage therapeutic composition providing effects of minimum loss of cartilage therapeutic agent due to combined use and solidification of chondrocyte components, thrombin and a fibrinogen matrix component, more complete cartilage regeneration effects by interaction between the fibrinogen matrix and cells, reduction of surgical operation time and utilization of an arthroscope, and a method of using the same.

### [Technical Solution]

In accordance with an aspect of the present invention, the above and other objects can be accomplished by the provision of a cartilage therapeutic composition comprising a mixture of components of chondrocytes isolated and expanded or differentiated from a host, thrombin and a fibrinogen matrix.

In accordance with another aspect of the present invention, there is provided a method of using a cartilage therapeutic composition, comprising:
preparing components of chondrocytes isolated and expanded or differentiated from a host;
preparing thrombin;
preparing a fibrinogen matrix;
treating a cartilage defect region; and
mixing the chondrocyte components, thrombin and fibrinogen matrix and injecting the resulting mixture into the cartilage defect region.

### [Description of the Drawings]

The above and other objects, features and other advantages of the present invention will be more clearly understood from the following detailed description taken in conjunction with the accompanying drawings, in which:
Fig. 1 is a graph showing strength of a matrix for transplanting chondrocytes in accordance with the present invention, with respect to an injection concentration of fibrinogen and thrombin;
Fig. 2 is a photograph of a matrix when fibrinogen was injected in an amount of 200 mg/mL (A), 160 mg/mL (B) and 20 mg/mL, respectively, in Fig. 1;

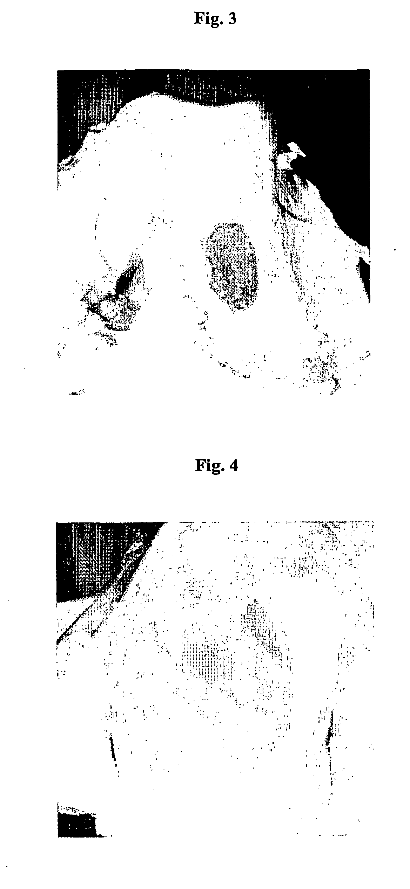

Fig. 3 is a photograph showing the trimmed cartilage defect region in which a matrix in accordance with the present invention was transplanted;
Fig. 4 is a photograph showing a transplanted site in which a matrix in accordance with the present invention is transplanted;
Fig. 5 is a photograph showing an injection state of a cartilage therapeutic composition after periosteum suture in accordance with another example of the present invention;
Fig. 6 is a photograph showing an injection state of a cartilage therapeutic composition after previously forming a connection hole at a cartilage defect region, in accordance with another example of the present invention;
Fig. 7 is a photograph showing chondrocyte differentiation patterns in a fibrinogen matrix in accordance with the present invention; and
Fig. 8 is a photograph showing chondrocyte differentiation patterns in a fibrinogen matrix containing 10% collagen and 10% hyaluronic acid in accordance with another example of the present invention.

### [Best Mode]

Now, the present invention will be described in more detail.

The present invention may improve, maintain and restore functions of tissues by using tissue engineering involving replacement of tissues and organs.

In accordance with the present invention using tissue engineering, provided is a cartilage therapeutic composition formed by mixing components of chondrocytes isolated and expanded or differentiated from a host such as human or animal, and thrombin and a fibrinogen matrix.

In addition, the present invention requires a matrix to which specific cells and growth factors may be included as a tissue replacement, and uses in vivo components as a matrix, such as constituents of extracellular matrix (ECM), and a natural polymer as a matrix replacement.

The interaction between the chondrocyte and matrix directly promotes cell adhesion, migration, growth, differentiation and apoptosis.

Further, such interaction regulates activity of cytokines and growth factors, and activates an intracellular signal transduction process resulting in rapid growth and restoration of tissues of interest.

Meanwhile, a method of using a cartilage therapeutic composition in accordance with the present invention involves preparing components of chondrocytes isolated and expanded or differentiated from humans or animals, and preparing thrombin that is used as a clotting factor.

This is followed by preparation of a fibrinogen matrix that is to be mixed with the chondrocyte components and thrombin, that is, components such as fibrinogen, collagen, hyaluronic acid, glycosaminoglycan (hereinafter, "GAG") and aprotinin.

In this connection, as the fibrinogen matrix necessarily containing fibrinogen, fibrinogen may be used alone in an appropriate concentration, or the fibrinogen matrix may be formed by mixing fibrinogen and other matrix components such as collagen, hyaluronic acid, GAG and aprotinin in a suitable ratio.

The aprotinin serves to inhibit degradation of fibrinogen by plasmin, thus enabling a shape of the cartilage therapeutic agent (a mixture of thrombin, chondrocyte components and fibrinogen) to be maintained in a predetermined form, for a longer period of time, when components of the cartilage therapeutic agent are mixed and solidified.

At this time, the cartilage defect region, to which the cartilage therapeutic agent will be transplanted, is first trimmed, and then a hole having a diameter of 2 to 3 mm and a depth of 3 to 5 mm is made inside a transplantation site for transplanting, followed by spraying thrombin.

Then, the cartilage therapeutic agent composed of a mixture of chondrocyte components, thrombin and a fibrinogen matrix is injected to the cartilage defect region to which thrombin was sprayed.

Subsequently, a high concentration of thrombin is sprayed for engrafting and shape maintenance of the cartilage therapeutic agent onto the upper side of the cartilage defect region to which the cartilage therapeutic agent was injected.

In the present invention, the chondrocyte components are prepared and used by separating chondrocytes from normal cartilage tissue using a suitable enzyme, and incubating the separated cells in a culture medium to obtain a culture containing more than 10⁶ cells/mL.

In addition, the fibrinogen is restored to its original state from lyophilized fibrinogen using conventional cell culture media such as DMEM (Dulbecco's Modified Eagle's Medium), Ham's F-12 medium, DMEM/F-12, IMDM (Iscove's Modified Dulbecco's Medium), McCoy's 5A Medium, MEM (Minimum Essential Medium), α-MEM, RPMI 1640 medium, Leibovitz's L-15 Medium, and Eagle's basal Medium.

The thrombin is also restored to its original state from lyophilized thrombin using the above-mentioned culture media including DMEM medium.

Further, the fibrinogen matrix is formed by mixing the fibrinogen with at least one of collagen, hyaluronic acid and GAG, and aprotinin in a predetermined concentration.

In this connection, fibrinogen is a substance present in the blood, which plays a role in clotting as it reacts with thrombin and then is converted into fibrin that is then clotted by CaCl₂ and a factor XIII added to thrombin, thus achieving a hemostatic function.

In addition, the culture medium for lysing the lyophilized fibrinogen may contain less than 10% of FBS (fetal bovine serum), FCS (fetal calf serum), calf serum, horse serum or human serum and in order to prevention infection with microorganisms, antibiotics such as penicillin G, kanamycin, gentamycin and streptomycin or antifungal agents such as amphotericin B and nystatin may be added thereto in an appropriate amount.

Additionally, as the culture medium for lysing the lyophilized fibrinogen, all the conventional medium for cell culture may be used including McCoys 5A medium, Eagle's basal medium, Glasgow minimum essential medium, Ham's F-12 medium, Iscove's modified Dulbecco's medium, Liebovitz' L-15 medium and RPMI 1640 medium.

In a specific embodiment of the fibrinogen matrix, the fibrinogen is used alone within a concentration of 20 mg/mL to 200 mg/mL, depending on conditions of a transplantation site and patient.

When present at a high concentration, the fibrinogen is relatively hard and forms a dense matrix, but leads to increased degradation time resulting in sensation of foreign matter in body after use thereof. In contrast, when it is present at a low concentration, degradation of the fibrinogen within tissue rapidly progresses, but a loose matrix is formed, thus resulting in difficulty of replacement with cartilage.

Further, the fibrinogen matrix may contain 0.01 mg/mL to 20 mg/mL of collagen, 0.1 mg/mL to 20 mg/mL of hyaluronic acid and 0.1 mg/mL to 20 mg/mL of GAG (glycosaminoglycan), and 1 to 3000 KIU/mL of aprotinin alone or in any combination thereof, in addition to 20 mg/mL to 200 mg/mL of fibrinogen.

The above-mentioned collagen, hyaluronic acid, GAG, and aprotinin are substances present in host animals, and even when they are administered over a predetermined concentration, effects on cells does not significantly vary but hardening time increases leading to retardation of surgical operation time.

Additionally, aprotinin is a substance that inhibits degradation of fibrin in vivo and thereby assists in a hemostatic function, and when the added amount of the aprotinin increases, the degradation rate of fibrin decreases. Therefore, it is possible to control a degradation rate of the matrix by adjusting the aprotinin concentration.

Generally, cartilage of vertebral animals consists of chondrocytes and a matrix. The matrix is largely composed of GAG containing collagen and hyaluronic acid and has no blood vessels thereby receiving nutrients from synovial fluid.

The main component of the synovial fluid is hyaluronic acid, and therefore, use of a mixture containing such a component provides excellent cartilage regeneration capabilities.

0.01 IU/mL to 50 IU/mL of the prepared thrombin is mixed in the cartilage therapeutic agent of the present invention and therefore, when mixing cartilage components with the fibrinogen matrix, the thrombin converts fibrinogen into fibrin.

Therefore, by adjusting the concentration of the thrombin added to the mixture of the cartilage therapeutic agent, it is possible to control a hardening rate of the cartilage therapeutic agent, and also to control physical properties of the fibrinogen matrix.

Where the concentration of thrombin is low, the matrix is composed of thick fibrous materials and has a large pore size. Therefore, it is advantageous to incorporate cells and physiologically active materials but this leads to retardation of hardening time thus impeding the achievement of desired objects.

Conversely, where the concentration of the thrombin is high, the matrix is composed of thin fibrous materials and has a small pore size. Therefore, it is advantageous to allow for easy ingrowth while blocking cell penetration, but this leads to rapid hardening time resulting in extremely low workability due to hardening of the matrix during mixing of respective components.

Therefore, the thrombin concentration is preferably within the range of 0.01 IU/mL to 50 IU/mL, and the injected thrombin hardens within 1 to 10 min. In addition, preferably, when the thrombin is mixed with the matrix of the present invention, the thrombin is used at a low concentration, and when it is used before or after injection of the composition, the thrombin is used at a high concentration.

As a result, it is possible to obtain a matrix having mechanical strength and flexibility suitable for regeneration of cartilage tissues.

It is also possible to easily modify the shape of the matrix such that it is adapted to the various possible configurations present in damaged cartilage region, thus enhancing the convenience of the associated surgical operation, and also allowing the matrix to be continuously positioned at a damaged region while maintaining a predetermined shape thereof after transplantation, leading to promotion of in vivo expansion and differentiation of the grafted chondrocytes.

In the cartilage defect region at which a composition for transplanting chondrocytes according to the present invention is to be transplanted, the defect region is first trimmed, zero to five holes having a diameter of 2 to 3 mm and depth of about 3 to 5 mm are made inside the transplanting site depending on the size of the affected part, followed by stanching, and then 100 IU/mL to 1000 IU/mL of high concentration thrombin is sprayed onto the surface of the affected part to increase adhesivity of cartilage therapeutic agent.

At this time, the thrombin serves to induce close binding between the cartilage therapeutic agent and the cartilage defect region to which the cartilage therapeutic agent is to be transplanted, thus increasing cartilage regeneration density while withstanding external impact.

In addition, a cartilage therapeutic composition is injected into a cartilage defect region of a human or animal patient.

Injection of the cartilage therapeutic composition is performed by slow injection to the perforated part of the cartilage defect region followed by injection to fill all the remaining defect parts.

Since the time required for conversion of fibrinogen into fibrin following mixing between fibrinogen and thrombin is concentration-dependent, the cartilage therapeutic agent is injected to the defect region within a suitable period of time between about 1 and 10 min.

Further, when transplanting the cartilage therapeutic composition into the cartilage defect region, 100 IU/mL to 1000 IU/mL of high concentration thrombin is sprayed onto the transplanted cartilage therapeutic composition composed of chondrocyte components, thrombin and fibrinogen, in order to assist in engraftment and shape maintenance thereof, thereby forming a protective film to protect cartilage therapeutic agent during cartilage regeneration.

The transplanted site is left for about 5 min such that it is completely hardened during conversion of fibrinogen into fibrin by action of thrombin.

### [Mode for Invention]

Now, the present invention will be described in more detail with reference to the following Examples. These examples are provided only for illustrating the present invention and should not be construed as limiting the scope and sprit of the present invention.

### EXAMPLES

### Experimental Example 1

In order to determine appropriate physicochemical properties of various matrices used in transplanting a cartilage therapeutic agent, commercially available lyophilized thrombin was lysed to a concentration of 2 IU/ml to 10 IU/mL.

2 IU/ml to 10 Iµ/mL of thrombin was added to convert fibrinogen, a main matrix, into fibrin.

Additionally, collagen, hyaluronic acid and GAG were, respectively, added to 0.01 mg/mL to 20 mg/mL, 0.1 mg/mL to 20 mg/mL and 0.1 mg/mL to 20 mg/mL to prepare the respective matrix.

Each matrix thus prepared was mixed and the resulting hardness, strength and bending strength thereof were measured.

First, 1 mL of fibrinogen having a concentration of 20 mg/mL to 200 mg/mL and 1 mL of thrombin having a concentration of 2 IU/mL to 10 IU/mL were mixed and the hardness and strength of the resulting respective composition were measured.

In addition, the practical cartilage therapeutic agent and the matrix having the above-mentioned concentration were mixed and then the strength of the resulting composition was measured.

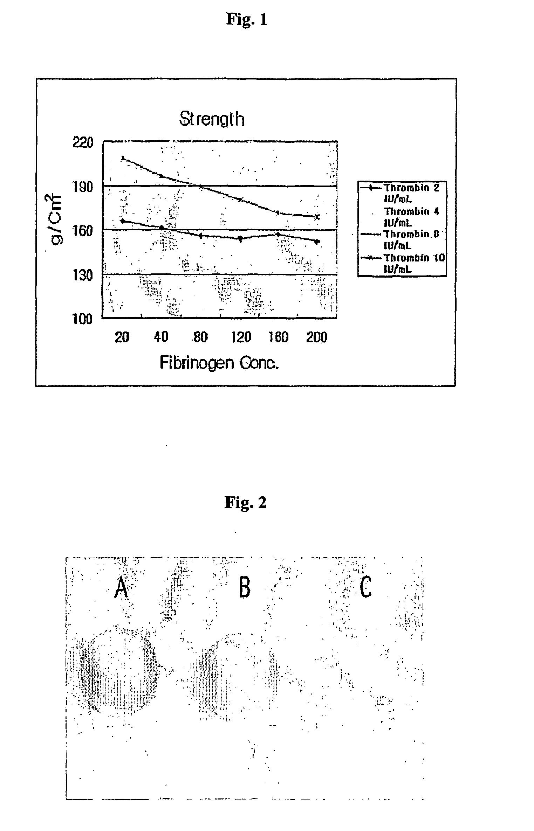

As a result, as shown in Fig. 1, through various clinical trials, when transplanting a cartilage therapeutic composition consisting of a chondrocyte component-thrombin-fibrinogen mixture, a suitable strength thereof was about 160 to 170 g/cm². Based on the results measured as described above, strength of the composition was obtained as follows: 166 g/cm² for 2 IU/mL of thrombin and 20 mg/mL of fibrinogen; 170 g/cm² and 165 g/cm² for 4 IU/mL of thrombin, and 160 mg/mL and 200 mg/mL of fibrinogen, respectively; 170 g/cm² and 166 g/cm² for 8 IU/mL of thrombin, and 160 mg/mL and 200 mg/mL of fibrinogen, respectively; and 171 g/cm² and 169 g/cm² for 10 IU/mL of thrombin, and 160 mg/mL and 200 mg/mL of fibrinogen, respectively. Consequently, suitable concentrations of fibrinogen and thrombin for transplantation and characteristics as shown in Fig. 2 were obtained.

Differences between physical properties with respect to the fibrinogen concentrations of the composition showed characteristics shown in photographs of Fig. 2 (A: 200 mg/mL, B: 160 mg/mL, and C: 20 mg/mL from left to right).

When measuring hardness of each composition in which each matrix, i.e., collagen, hyaluronic acid and GAG were mixed with appropriate concentrations of fibrinogen and thrombin, the measured strength of the cartilage therapeutic composition consisting of a chondrocyte component-thrombin-fibrinogen mixture, to which hyaluronic acid, collagen and GAG were added, respectively, was about 154 to 159 g/cm², with decrease of about 1 to 6 g/cm² in strength, as compared to the above-measured results.

Such decrease in strength may be controlled by adjusting the concentrations of matrix components such as collagen, hyaluronic acid and GAG, or using any combination thereof.

In addition, since the composition of the matrix varies depending on conditions and characteristics of the patient, the above-mentioned range of fibrinogen and thrombin concentrations is used in combination with matrix components such as collagen, hyaluronic acid and GAG.

Further, cartilage generation effects can be obtained by addition of collagen or hyaluronic acid in the case of elderly patients, and by use of composition in combination with GAG or hyaluronic acid in the case of young patients suffering from severe trauma, e.g. due to a traffic accident.

### Experimental Example 2

Polymerization time required to impart the injected fibrinogen and thrombin with strength of 160 to 170 g/cm², suitable for use as the cartilage therapeutic agent, is shown in Table 1.

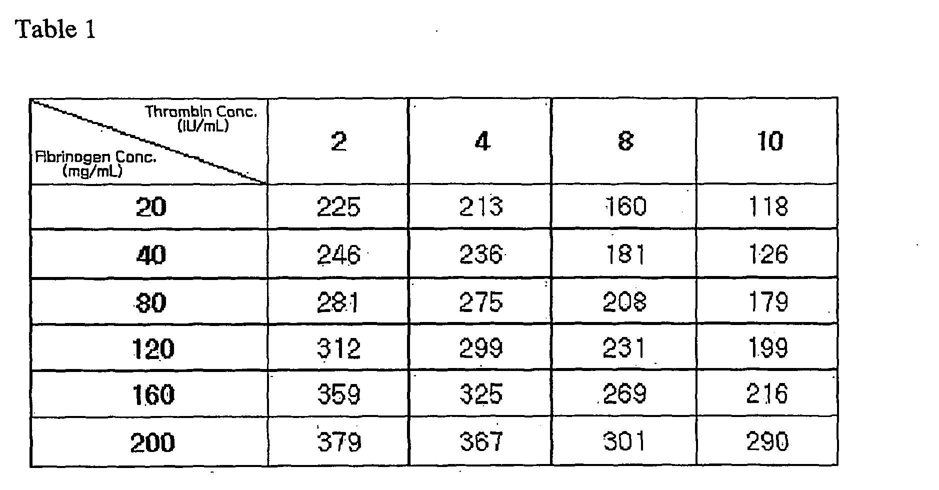

That is, a low concentration of thrombin exhibited a slow polymerization time and formed a thin fibrous structure having a pore size of more than about 5 µm, while a high concentration of thrombin exhibited a rapid polymerization time and formed a structure having a small pore size of less than about 1 µm.

Additionally, it can be seen that the composition composed of thrombin and fibrinogen took 2 to 7 minutes to exhibit strength of 150 to 180 g/cm², suitable for use in the cartilage therapeutic agent.

### Example 1

### Injection of cartilage therapeutic composition by arthroscope

First, cartilage tissue was collected from an animal or human host and chondrocytes were isolated from the tissue. Then, the isolated cells were cultured to prepare a cartilage therapeutic agent consisting of 0.3 to 0.5 mL of DMEM and 9x10⁶ to 1.5x10⁷ chondrocytes.

Then, as a commercially available medical product, a fibrin sealant was prepared and a DMEM was added to a vial containing lyophilized fibrinogen such that fibrinogen was lysed to a concentration of 100 mg/mL to 200 mg/mL.

Additionally, lyophilized thrombin was lysed to a concentration of 500 IU/mL and then the resulting thrombin solution was added to the cartilage therapeutic agent in the concentration of 1 IU/mL to 10 IU/mL.

After completion of the above-mentioned process, a cartilage defect region of the patient's knee was ready for transplantation by trimming the cartilage defect region using the arthroscope.

Next, the damaged cartilage region of the patient was cleanly trimmed using surgical instruments and then three to five holes having a diameter of 3 mm and depth of 5 mm were made within the damaged region using a surgical drill, depending on the size of the defect area.

Then, debris of cartilage and bone was removed from the defect region and stanched to finish necessary steps for transplantation.

After completion of necessary steps for transplantation, chondrocyte components, thrombin and a matrix containing at least fibrinogen were mixed to prepare a cartilage therapeutic agent.

In this connection, since fibrinogen reacts with thrombin followed by conversion into fibrin that in turn clots, the cartilage therapeutic agent should be transplanted within 5 minutes into a transplantation site.

Subsequently, 500 IU/mL of the previously prepared thrombin solution was sprayed at the transplantation site of the cartilage therapeutic composition using a spray tip and then was gently wiped with gauze.

Then, the cartilage therapeutic composition, composed of a mixture of chondrocyte components, thrombin and fibrinogen, was slowly injected using a syringe through the previously perforated holes in the affected part to the entire area sequentially.

Injection of the composition should be rapidly performed without formation of bubbles, and immediately after completion of injection, 500 IU/mL of high concentration thrombin solution was equally sprayed using the spray tip.

### Example 2

### Transplantation of cartilage therapeutic agent in which collagen was added to a composition of chondrocyte components, thrombin and fibrinogen mixture, under arthroscope

As described above, chondrocyte components, thrombin and fibrinogen were prepared.

In order to reinforce elasticity and to obtain cartilage regeneration effects in a patient exhibiting decreased articular elasticity, a fibrinogen matrix mixed with collagen may be used for transplantation.

The fibrinogen matrix is made up of fibrinogen, and collagen, hyaluronic acid and GAG added thereto. Upon combined use of fibrinogen and collagen, for an 50-year old male patient, a tenth or a fifth concentration of collagen may be used alone or in an admixture, relative to the fibrinogen matrix concentration, even though collagen concentration varies depending on conditions of the patient.

For example, in the case of the matrix for transplantation into a 52-year old male patient, fibrinogen and collagen were prepared to 80 mg/mL to 160 mg/mL concentration and 10 mg/mL to 20 mg/mL concentration, respectively. Then, 0.3 mL of fibrinogen and 0.1 mL of collagen were mixed to prepare a fibrinogen matrix for transplantation.

Next, a hole for inserting an arthroscope was made at an affected part, as in Example 1, and the arthroscope was then inserted therethrough to cleanly trim the transplantation site. Then, transplantation was ready by making about 4 holes having a diameter of 3 mm and depth of 5 mm at the affected part (lesion size of patient is about 4 cm²).

Subsequently, 1 IU/mL to 10 IU/mL of thrombin was added to chondrocyte components, and then, the cartilage therapeutic composition, in which the fibrinogen matrix containing a mixture of chondrocyte components and collagen was mixed, was injected and filled into the cartilage defect region.

Subsequent processes are the same as Example 1.

Meanwhile, when transplanting the fibrinogen matrix formed by adding other mixtures to the fibrinogen, kinds of the mixtures added to the fibrinogen may vary depending on conditions and age of the patient. For older patients, matrix components such as collagen and hyaluronic acid may be mixed and used with the cartilage therapeutic agent.

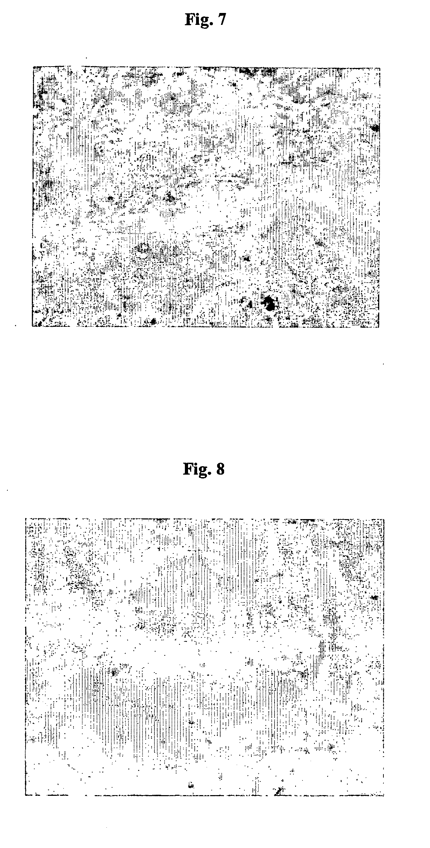

Fig. 7 shows the differentiation pattern of chondrocytes in the fibrinogen matrix as described above. Differentiation patterns of chondrocytes in the fibrinogen matrix containing 10% collagen and 10% hyaluronic acid are shown in Fig. 8.

### Example 3

### Injection of a cartilage therapeutic composition using periosteum

First, cartilage tissue was collected from a patient and chondrocytes were isolated from the tissue and cultured. This was followed by preparation of a cartilage therapeutic agent consisting of more than about 1.2 x 10⁷ chondrocytes and 0.4 mL of DMEM.

Then, as a commercially available medical product, a fibrin sealant was prepared and DMEM was added to a vial containing lyophilized fibrinogen such that the fibrinogen was dissolved to a concentration of 50 mg/mL to 80 mg/mL.

Additionally, DMEM was added to a vial containing lyophilized thrombin such that the thrombin was dissolved to a concentration of 500 IU/mL.

Then, a site for transplanting a cartilage therapeutic composition was smoothly trimmed using a surgical instrument.

After completion of the above-mentioned process, a periosteum was collected from the patient's knee and then was carefully sutured to the transplantation site.

After completion of suturing, a sealant was applied to the sutured site.

Then, it was determined whether watertight integrity of the sutured periosteum was complete or not.

Next, to the previously prepared chondrocyte components was added a solution of dissolved thrombin in the concentration of 1 IU/mL to 10 IU/mL, followed by good mixing.

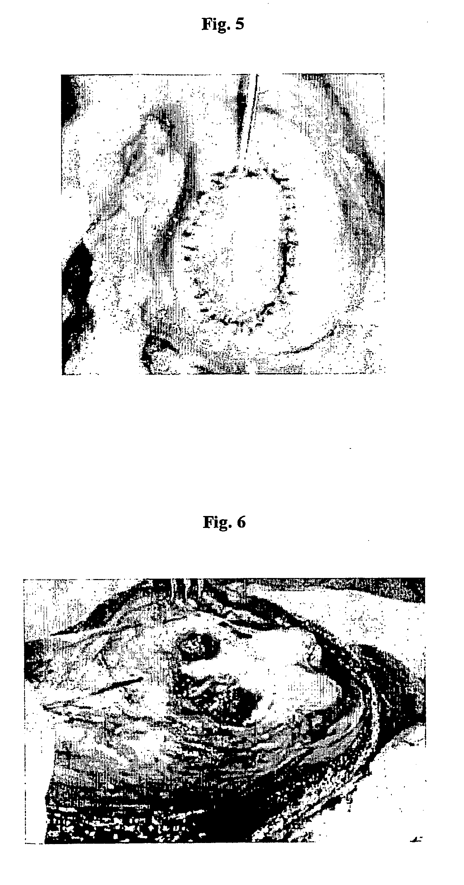

Next, 0.4 mL of chondrocytes components mixed with thrombin was mixed well with 0.4 mL of a fibrinogen solution to form a transplantation composition. The resulting composition was injected into the periosteum of the affected part using a syringe, as shown in Fig. 5.

In this connection, since fibrinogen reacts with thrombin followed by conversion into fibrin that in turn clots, injection should be performed rapidly.

### Example 4

### Injection of a composition made up of a mixture of a fibrinogen matrix in which hyaluronic acid and GAG were added to fibrinogen and cellular components to which thrombin was added

A portion of cartilage tissue was collected from a 27-year old male suffering from cartilage damage due to a traffic accident and cultured to prepare about 3 x 10⁷ cells/mL.

As constituents of a fibrinogen matrix, 50 mg/mL to 100 mg/mL of fibrinogen, 10 mg/mL of hyaluronic acid and 5 mg/mL to 10 mg/mL of GAG were prepared.

Since the lesion on the patient due to cartilage damage resulting from the traffic accident had a size of 4 to 8 cm², 0.5 mL of 50 mg/mL to 100 mg/mL fibrinogen, 0.2 mL of 10 mg/mL hyaluronic acid and 0.2 mL of 5 mg/mL to 10 mg/mL GAG were mixed.

A site for transplanting the cartilage therapeutic agent was cleanly trimmed and two to five holes having a diameter of 3 mm and depth of 5 mm were made, depending on the size of damaged area.

Then, chondrocyte components mixed with 5 IU/mL of thrombin, and a three-component mixed fibrinogen matrix was prepared.

Subsequently, 500 IU/mL of high concentration thrombin was equally sprayed onto the transplantation site using a spray tip. Then, 0.9 mL of chondrocyte components and the three-component-mixed fibrinogen matrix were mixed.

The composition made up of the mixed chondrocyte componentfibrinogen matrix (hyaluronic acid+GAG) was slowly applied via the holes of the transplantation site.

Again, 500 IU/mL of high concentration thrombin was sprayed onto the transplanted site and left for about 5 min. The transplantation process was finished with completion of hardening.

In this connection, since young patient exhibits good elasticity of cartilage tissues but has less smoothness of cartilage part due to trauma, use of hyaluronic acid or GAG may alleviate such disadvantage thus promoting easy cartilage regeneration.

### [Industrial Applicability]

As described above, in accordance with the present invention, provided are advantages such as prevention of cartilage therapeutic agent leakage, a simplified surgical process for maintaining a predetermined shape, and capability of maintaining excellent cartilage generation due to combination transplantation of in vivo matrix components.

Further, provided are effects such as reduction of surgical operation time when periosteum is not sutured, a simplified surgical process due to utilization of an arthroscope, capability of maintaining excellent cartilage regeneration due to combinational transplantation with in vivo matrix components, and no need for knee incision.

Although the preferred embodiments of the present invention have been disclosed for illustrative purposes, those skilled in the art will appreciate that various modifications, additions and substitutions are possible

## Claims

1. A cartilage therapeutic composition comprising a mixture of components of chondrocytes isolated and expanded or differentiated from a host, and thrombin and a fibrinogen matrix containing fibrinogen,
wherein the thrombin is used in the amount of 0.01 to 50 IU/mL,
wherein the fibrinogen matrix contains 0.01 mg/mL to 20 mg/mL of collagen and 0.1 mg/mL to 20 mg/mL of hyaluronic acid,
wherein the fibrinogen matrix contains 20 mg/mL to 200 mg/mL of fibrinogen.

2. The composition as set forth in claim 1, wherein the cell components are prepared by separating cells from a normal cartilage tissue isolated from the host using an enzyme, and incubating cells in a culture medium to obtain a culture containing more than 10⁶ cells/mL.

3. The composition as set forth in claim 1, wherein the fibrinogen matrix contains at least one selected from antibiotics such as penicillin G and streptomycin and antifungal agents such as kanamycin, amphotericin B, nystatin and gentamycin.

## Patentansprüche

1. Therapeutische Knorpelzusammensetzung, bestehend aus ein Gemisch von Komponenten, umfassend Chondrocyten, welche aus einem Wirt isoliert und dann vermehrt bzw. differenziert wurden, sowie Thrombin und eine Fibrinogen enthaltende Fibrinogen-Matrix,
wobei das Thrombin in einer Menge von 0,01 bis 50 IU/ml verwendet wird,
wobei die Fibrinogen-Matrix 0,01 mg/ml bis 20 mg/ml Kollagen und 0,1 mg/ml bis 20 mg/ml Hyaluronsäure enthält, und
wobei die Fibrinogen-Matrix 20 mg/ml bis 200 mg/ml Fibrinogen enthält.

2. Zusammensetzung nach Anspruch 1, wobei die Zellkomponenten durch die Vereinzelung von Zellen eines normalen Knorpelgewebes, welches aus einem Wirt isoliert wurde, unter Verwendung eines Enzyms und die Inkubation der Zellen in einem Kulturmedium, um eine Kultur zu erhalten, welche mehr als 10⁶ Zellen/ml enthält, bereitgestellt werden.

3. Zusammensetzung nach Anspruch 1, wobei die Fibrinogen-Matrix mindestens eine Verbindung gewählt aus Antibiotika wie Penicillin G und Streptomycin sowie antimykotischen Mitteln wie Kanamycin, Amphotericin B, Nystatin und Gentamycin enthält.

## Revendications

1. Composition thérapeutique pour cartilage, comprenant un mélange de composants de chondrocytes isolés et expansés ou différenciés issus d'un hôte, ainsi que de la thrombine et une matrice à fibrinogène qui contient du fibrinogène, dans laquelle composition :
- la thrombine est utilisée à raison de 0,01 à 50 UI/mL,
- la matrice à fibrinogène contient de 0,01 à 20 mg/mL de collagène et de 0,1 à 20 mg/mL d'acide hyaluronique,
- et la matrice à fibrinogène contient de 20 à 200 mg/mL de fibrinogène.

2. Composition conforme à la revendication 1, pour laquelle on a préparé les composants cellulaires en séparant des cellules, à l'aide d'une enzyme, d'un tissu cartilagineux normal isolé provenant de l'hôte, et en faisant incuber ces cellules dans un milieu de culture, de manière à obtenir une culture contenant plus de 10⁶ cellules par millilitre.

3. Composition conforme à la revendication 1, dans laquelle la matrice à fibrinogène contient au moins un agent choisi parmi les antibiotiques, comme de la pénicilline G ou de la streptomycine, et les antifongiques, comme de la kanamycine, de l'amphotéricine B, de la nystatine ou de la gentamycine.
